# EUROPÄISCHE PATENTSCHRIFT

(11) **EP 1 123 917 B1**
(45) Veröffentlichungstag und Bekanntmachung des Hinweises auf die Patenterteilung: **06.05.2004**
(21) Anmeldenummer: 01109422.4
(22) Anmeldetag: 20.02.1998
(51) Int. Cl.: C07C 209/68, C07C 209/78, C07C 211/50, C07C 209/54

(54) **Verfahren zur Herstellung von aromatischen Polyamingemischen**
Process for preparing aromatic polyamine mixtures
Procédé pour la préparation de mélanges de polyamines aromatiques

(30) Priorität: 24.02.1997 DE 19707255
(43) Veröffentlichungstag der Anmeldung: 16.08.2001
(62) Teilanmeldung aus: 98913570.2
(73) Patentinhaber: BASF AKTIENGESELLSCHAFT, 67056 Ludwigshafen (DE)
(72) Erfinder: Becker, Rainer, Dr., 67098 Bad Dürkheim (DE); Eller, Karsten, Dr., 67061 Ludwigshafen (DE); Langensiepen, Hans-Werner, Dr., 67240 Bobenheim-Roxheim (DE); Hesse, Michael, Dr., 67549 Worms (DE)
(74) Vertreter: Isenbruck, Günter, Dr.

(56) Entgegenhaltungen:
- EP-A- 0 264 744
- DE-A- 1 493 431
- GB-A- 1 207 377
- US-A- 4 039 580
- US-A- 4 039 581
- US-A- 4 071 558
- US-A- 4 294 987

## Beschreibung

Die Erfindung betrifft ein Verfahren zur Herstellung von aromatischen Polyamingemischen aus gegebenenfalls substituiertem Anilin und Formaldehyd oder Formaldehyd-Vorläufern oder einem Kondensationsprodukt dieser Verbindungen.

Diaminodiarylmethane, die gegebenenfalls substituiert sind, sind wertvolle Vorprodukte vor allem für die Herstellung von Kunststoffen. Vor allem das unsubstituierte Diaminodiphenylmethan (häufig als Methylendianilin, MDA bezeichnet) wird technisch in sehr großen Mengen hergestellt und größtenteils, nach Phosgenierung zu Methylendiphenyldiisocyanat (MDI), für die Herstellung von Polyurethanen eingesetzt. Dabei wird vorzugsweise das 4,4'-Isomere verwendet, jedoch entstehen in den bekannten Herstellungsverfahren auch die 2,4'- und 2,2'-Isomeren. Zudem werden höherkondensierte, mehrkernige Verbindungen gebildet. Die Herstellung erfolgt in der Regel aus Anilin und Formaldehyd in Gegenwart von Katalysatoren.

In den technisch ausgeübten Verfahren werden anorganische Säuren in gelöster Form als homogene Katalysatoren eingesetzt. Bevorzugt wird dabei wäßrige Salzsäure verwendet. Diese Reaktionsführung führt durch die Aufarbeitung bedingt zum Verbrauch äquimolarer Mengen an Basen, da die Säuren bei der Isolierung der gewünschten Polyamine noch neutralisiert werden müssen. Dieser Vorgang ist daher zwangsläufig mit einem Anfall entsprechend hoher Salzmengen verbunden, die entsorgt werden müssen oder aufwendig zurückgeführt werden. Ferner ist die mit der Verwendung wäßriger Säuren verbundene Korrosionsproblematik ein wesentlicher Nachteil dieses Verfahrens.

Aus diesem Grunde gab es zahlreiche Überlegungen und Versuche, um die wäßrigen Homogenkatalysatoren durch saure Heterogenkontakte zu ersetzen. Neben sauren Ionentauschern wurde die Verwendung von sauren synthetischen oder natürlichen Silicium- oder Aluminiumoxiden, wie Zeolithen oder Tonmineralien vorgeschlagen. In DE-A-1 230 033, DE-A-1 493 431, US 4,071,558, US 4,039,580, US 4,039,581 und US 4,294,987 sind entsprechende Katalysatoren beschrieben.

Gemäß US 4,294,987 wird in einem derartigen Verfahren die Kondensation in Gegenwart einer starken wäßrigen Säure durchgeführt, wonach durch Lösungsmittelextraktion die Säure entfernt wird. Die Umlagerung wird wiederum in Gegenwart von starker Säure, die in geringerer Menge eingesetzt wird, durchgeführt. Auch Diatomeenerde, Ton oder Zeolithe können in dieser Stufe eingesetzt werden.

Gemäß DE-A-1 230 033 wird siliciumhaltiger Ton, ein synthetischer Siliciumdioxid-Aluminiumoxid-Katalysator oder ein Magnesiumoxid-Aluminiumoxid-Katalysator eingesetzt.

Gemäß DE-A-1 493 431 wird Siliciumdioxid, Siliciumdioxid/Aluminiumoxid oder säurebehandeltes Aluminiumoxid als Katalysator eingesetzt. Bevorzugt wird Kieselgel oder bentonitartiger Ton, der Siliciumdioxid und Aluminiumoxid enthält und vorzugsweise säureaktiviert ist, eingesetzt. Neben den gewünschten Diaminodiarylmethanen fallen jedoch höhere Kondensationsprodukte in erheblichem Umfang an.

Gemäß US 4,071,558 wird ein mit Säure aktivierter Tonkatalysator, ein Siliciumdioxid-Aluminiumoxid-Crack-Katalysator oder ein Siliciumdioxid-Magnesiumoxid-Katalysator eingesetzt. Auch hier fallen bei Verwendung des Tonkatalysators größere Mengen an 2,4'-Isomeren und höhermolekularen Produkten an.

Gemäß US 4,039,580, US 4,039,581 und US 4,294,987 wird ein Gemisch aus Diaminodiarylmethanen und höherkondensierten Oligomeren in aufwendigen zweistufigen Verfahren erhalten. Neben wäßrigen Säuren werden auch Diatomeenerde, Tone oder Zeolithe eingesetzt.

Gemäß US 4,294,987 wird die Kondensation in Gegenwart einer starken wäßrigen Säure durchgeführt, wonach durch Lösungsmittelextraktion die Säure entfernt wird. Die Umlagerung wird wiederum in Gegenwart von starker Säure, die in geringerer Menge eingesetzt wird, durchgeführt. Auch Diatomeenerde, Tone oder Zeolithe können in dieser Stufe eingesetzt werden.

Gemäß US 4,039,580 wird eine Kondensation von Anilin und Formaldehyd in Abwesenheit eines Katalysators ausgeführt und das Kondensationsprodukt in Gegenwart von Diatomeenerde, Tonen oder Zeolithen umgesetzt. Der Diamingehalt im Produkt beträgt 44 bis 50 %. In der US 4,039,581 sind ähnliche Umsetzungen beschrieben. Technisch haben sich diese Katalysatoren jedoch aufgrund der hohen Preise, der nicht ausreichenden Aktivitäten oder mangelnden Katalysatorstandzeiten nicht durchsetzen können. Hohe Anteile an 4,4'-Isomeren und sehr niedrige Gehalte an höherkondensierten Produkten sind in diesen Verfahren jedoch nicht zugänglich. Es fallen immer größere Anteile an 2,4'- bzw. 2,2'- Isomeren an.

Es besteht somit weiterhin die Nachfrage nach heterogenen Katalysatoren, die kostengünstig sind, eine hohe Aktivität und lange Standzeit aufweisen, umweltverträglich sind und zu hohen Ausbeuten an 4,4'-Isomeren bei geringen Mengen an höherkondensierten Produkten führen.

Aufgabe der vorliegenden Erfindung ist die Bereitstellung von Katalysatoren, die die vorgenannten Eigenschaften aufweisen, sowie eines Verfahrens zur Herstellung von aromatischen Polyamingemischen.

Erfindungsgemäß wird die Aufgabe gelöst durch ein Verfahren zur Herstellung von aromatischen Polyamingemischen, die Verbindungen der allgemeinen Formel (I) enthalten,

H₂N-A-CH₂-B-NH₂ (I)

in der A und B 1,4-Phenylenreste sind, die jeweils unabhängig 1 bis 4 Substituenten,ausgewählt aus C₁₋₂₀-Alkylresten und Halogenatomen, aufweisen können,
durch Umsetzung einer Verbindung der allgemeinen Formel (IV)

H-A-NH-CH₂-HN-B-H (IV)

und/oder einer Verbindung der allgemeinen Formel (V)

H-A-NH-CH₂-B-NH₂ (V)

wobei A und B wie vorstehend substituiert sind,
bei einer Temperatur im Bereich von 20°C bis 200°C in Gegenwart eines heterogenen anorganischen Katalysators, der ausgewählt ist aus einem Ton, der mit mindestens einem Oxid von Elementen der Gruppen 2 bis 13 oder der Lanthaniden des Periodensystems der Elemente durch Imprägnierung oder über einen Ionenaustausch dotiert ist und säureaktiviert sein kann oder einem oder mehreren gegebenenfalls säureaktivierten Schichtsilikaten, die eine Acidität unterhalb von pKₛ = 1,5 von mehr als 0,05 mmol/g Katalysator besitzen.

Dabei kann die Verbindung der allgemeinen Formeln (IV) und/oder (V) durch Umsetzung von gegebenenfalls am aromatischen Kern in o- oder m-Position mit 1 bis 4 Substituenten, ausgewählt aus C₁₋₂₀-Alkylresten oder Halogenatomen, substituiertem Anilin mit Formaldehyd oder Formaldehyd-Vorläufern erhalten werden.

Zudem wird die Aufgabe gelöst durch ein Verfahren zur Herstellung von aromatischen Polyamingemischen, die Verbindungen der allgemeinen Formel (I) enthalten,

H₂N-A-CH₂-B-NH₂ (I)

in der A und B 1,4-Phenylenreste sind, die jeweils unabhängig 1 bis 4 Substituenten, ausgewählt aus C₁₋₂₀-Alkylresten und Halogenatomen, aufweisen können,
durch Umsetzung von gegebenenfalls am aromatischen Kern in o- oder m-Position mit 1 bis 4 Substituenten, ausgewählt aus C₁₋₂₀-Alkylresten und Halogenatomen, substituierten Anilinen mit Formaldehyd oder Formaldehyd-Vorläufern bei einer Temperatur im Bereich von 20°C bis 200°C in Gegenwart eines heterogenen anorganischen Katalysators, der ausgewählt ist aus einem Ton, der mit mindestens einem Oxid von Elementen der Gruppen 2. bis 13 oder der Lanthaniden des Periodensystems der Elemente durch Imprägnierung oder über einen Ionenaustausch dotiert ist und säureaktiviert sein kann, oder einem oder mehreren gegebenenfalls säureaktivierten Schichtsilikaten, die eine Acidität unterhalb von pKₛ = 1,5 von mehr als 0,05 mmol/g Katalysator besitzen.

### Ton-Katalysator

Die Gruppeneinteilung des Periodensystems folgt der neuen Notation, vergleiche Cotton and Wilkinson, Advanced Inorganic Chemistry, 5. Auflage, John Wiley & Sons.

Als Tone können für die Katalysatoren alle di- oder trioktaedrischen Vertreter verwendet werden, beispielsweise Kaolin, Talk, Pyrophylit, Smectite wie Hectorit oder Montmorillonit, Vermiculit, Sepiolit oder Attapulgit. Bevorzugt sind Saponit, Hectorit, Montmorillonit, Sepiolit und Attapulgit, besonders bevorzugt ist Montmorillonit. Die Tone können entweder natürlichen Ursprungs oder synthetisch sein.

Die Tone können entweder in ihrer natürlich anfallenden Form oder nach einer vorherigen Säureaktivierung durch Imprägnierung oder über einen Ionenaustausch dotiert werden. Je nachdem enthalten die Tone somit vor der Dotierung unterschiedliche Mengen an Alkali- und Erdalkaliionen sowie die in natürlichen Tonen immer vorhandenen Verunreinigungen wie Eisenionen. Die Säureaktivierung kann mit verschiedenen Säuren erfolgen, bevorzugt sind die üblichen Mineralsäuren wie Salzsäure, Schwefelsäure oder Phosphorsäure. Besonders bevorzugt ist Salzsäure. Säureaktivierte Schichtsilikate sind im Handel unter den Namen Bleicherden oder Fuller-Erden erhältlich.

Die Oxide der Gruppen 2 bis 13 oder der Lanthaniden werden durch Imprägnierung oder über einen Ionenaustausch auf die Tone aufgebracht. Man kann beispielsweise den getrockneten Ton mit einer Lösung der aufzubringenden Metalle in Form ihrer Nitrate oder Acetate versetzen, die in ihrer Menge so berechnet sind, daß die aufgenommene Flüssigkeitsmenge genau den gewünschten Gehalt der Metallnitrate oder -acetate hat. Durch Trocknung und Kalzinierung werden die Nitrate oder Acetate in die gewünschten Oxide umgewandelt. Andere verwendbare Salze sind beispielsweise Oxalate oder Citrate, es können aber prinzipiell alle löslichen Salze eingesetzt werden, die sich bei den nachfolgenden Schritten der Katalysatorpräparation komplett oder teilweise zu den gewünschten Oxiden umwandeln. Sofern die Oxide selber löslich sind, können diese auch direkt verwendet werden.

Statt durch eine Imprägnierung in der oben beschriebenen Weise kann die Dotierung auch über einen Ionenaustausch erfolgen. Hierzu wird der Ton als Pulver in Suspension oder als Formkörper in einem Umlaufreaktor vorgelegt und mit einer Lösung in Kontakt gebracht, die die aufzubringenden Metalle in ionischer Form enthält. Die Metallionen werden dann einen Teil der Alkali-, Erdalkali- oder Wasserstoffionen des Tones ersetzen. Nach einer anschließenden eventuellen Waschung erfolgt wieder eine Trocknung und gegebenenfalls eine Kalzinierung. Für den Ionenaustausch können alle löslichen Metallverbindungen verwendet werden, die keine für die Katalyse negativen Verunreinigungen nach der Waschung, Trocknung oder Kalzinierung hinterlassen. Üblicherweise können sie Chloride, Nitrate oder Acetate der erfindungsgemäßen Metalle sein.

Die Trocknung der imprägnierten oder dotierten Schichtsilikate erfolgt zweckmäßigerweise bei Normaldruck und Temperaturen von 80 - 200°C, bevorzugt 100 - 150°C, für eine bis 20 Stunden. Es kann aber ebenso bei reduziertem Druck und niedrigeren Temerpaturen gearbeitet werden. Die Kalzinierung der getrockneten Katalysatoren erfolgt bei 150 - 600°C, bevorzugt 170 - 500°C, für einen Zeitraum von 0,5 bis 12 Stunden, bevorzugt eine bis 5 Stunden.

Die Menge an aufgebrachtem Oxid variiert in Abhängigkeit vom verwendeten Oxid oder der verwendeten Oxidmischung sowie dem eingesetzten Amin. Der optimale Gehalt kann jedoch leicht durch eine Konzentrationsreihe empirisch ermittelt werden. Erfindungsgemäß können bereits sehr niedrige Gehalte von 0,1 Gew.-%, berechnet als Metalloxid und bezogen auf das Gesamtgewicht des Katalysators, einen positiven katalytischen Effekt haben, bei sehr hohen Gehalten von über 20 Gew.-% wird aber keine weitere Steigerung der Aktivität und Selektivität mehr möglich sein. Bevorzugt sind daher Gehalte von 0,2 bis 10 Gew.-%, besonders bevorzugt von 0,3 bis 7 Gew.-%. Die Angabe des Gehaltes erfolgt pauschal in Gew.-% des stabilsten Oxides des jeweiligen Elements für ein bei 900°C geglühtes Schichtsilikat, es ist aber, da die genaue Umgebung der Metallionen im fertigen Katalysator nicht näher bekannt ist, ebenso möglich, daß das Schichtsilikat als Gegenion fungiert und/oder daß noch Reste des zur Aufbringung verwendeten Anions im Katalysator verbleiben.

Von den Oxiden der Gruppen 2 bis 13 oder der Lanthaniden sind die Oxide der Gruppen 2 bis 4, 7, 13 oder der Lanthaniden bevorzugt. Besonders bevorzugt sind Oxide des Bariums oder Strontiums aus der Gruppe 2, Lanthanoxid aus der Gruppe 3, Hafniumoxid aus der Gruppe 4, Rheniumoxid aus der Gruppe 7, Indiumoxid aus der Gruppe 13 sowie die Lanthaniden.

### Schichtsilikat-Katalysator

Es wurde erfindungsgemäß gefunden, daß nur bei Verwendung von bestimmten Schichtsilikaten genügend hohe Ausbeuten der gewünschten 4,4'-Diaminodiarylmethane erhalten werden. Bei nicht erfindungsgemäßen Schichtsilikaten erfolgt zwar ebenfalls die Bildung der Polyamingemische, aber erstens sehr viel langsamer und zweitens mit den in den vorstehend beschriebenen Patenten angegebenen Nachteilen wie geringer 4,4'-Selektivität und Bildung höherkondensierter Verbindungen.

Katalysatoren, die diese Mängel nicht besitzen, zeichnen sich dadurch aus, daß sie bei einer Titration gegen Hammett-Indikatoren, speziell den Hammett-Indikator 4-Phenylazo-diphenylamin (pKₛ=1,5), wie in Tanabe et al., New Solid Acids and Bases, Stud. Surf. Sci. Catal. 51, 1989, Chapter 2, sowie Benesi, J. Phys. Chem. 61, 1957, S. 970 - 973, näher beschrieben, eine Acidität von mehr als 0,05 mmol Butylamin pro Gramm Katalysator aufweisen. Zur Bestimmung der Acidität wird der getrocknete Katalysator in einem inerten aprotischen Lösungsmittel wie Toluol suspendiert und der Indikator mit dem definierten pKₛ-Wert von 1,5 sowie n-Butylamin oder eine ähnliche Base zugesetzt. Da die Reaktion der Feststoffsäure mit der Base langsam erfolgt, kann nicht in der üblichen Weise mittels einer Bürette titriert werden, sondern es werden in mehreren Ansätzen steigende Mengen an Base zugesetzt, über Nacht geschüttelt, und es wird am nächsten Morgen nach Einstellung des Gleichgewichtes überprüft, bei welchen Basenmengen der Umschlag erfolgt ist. Es ergibt sich so eine quantitative Menge an Aciditätszentren in mmol Base pro Gramm Feststoffsäure unterhalb des pKₛ-Wertes des Indikators von 1,5.

Als Schichtsilikate kommen für die Katalysatoren alle di- oder trioktaedrischen Vertreter in Frage, wie Kaolin, Talk, Pyrophylit, Smectite wie Hectorit oder Montmorillonit, Vermiculit, Muscovit, Sepiolit oder Attapulgit. Bevorzugt sind Saponit, Hectorit, Montmorillonit, Sepiolit und Attapulgit. Besonders bevorzugt sind Montmorillonit, Sepiolit und Attapulgit. Die Tone können entweder natürlichen Ursprungs oder synthetisch sein. Für die Katalyse ist es unerheblich, ob es sich um ein einzelnes definiertes Schichtsilikat handelt oder um ein Gemisch verschiedener Schichtsilikate. Insbesondere bei Verwendung natürlich vorkommender Schichtsilikate sind häufig verschiedene Schichtsilikate gleichzeitig anwesend.

Die Schichtsilikate können entweder bereits in ihrer natürlich anfallenden Form oder nach einer vorherigen Säureaktivierung eine genügende Acidität unterhalb von pKₛ=1,5 aufweisen. Sie enthalten somit unterschiedliche Mengen an Alkali- und Erdalkalimetallionen sowie die in natürlichen Tonen immer vorhandenen Verunreinigungen wie Eisenionen. Falls eine Säureaktivierung zur Erzielung einer genügend hohen Acidität notwendig ist, kann diese mit verschiedenen Säuren erfolgen. Bevorzugt sind die üblichen Mineralsäuren wie Salzsäure, Schwefelsäure oder Phosphorsäure. Besonders bevorzugt ist Salzsäure. Säureaktivierte Schichtsilikate sind im Handel unter den Namen Bleicherden oder Fuller-Erden erhältlich.

Die Katalysatoren können pulverförmig oder in Form von Formkörpern, wie Strängen, Granulaten, Tabletten, Pellets, Kugeln eingesetzt werden. Entsprechend kann die Umsetzung in Suspensionsfahrweise oder mit Festbettkatalysatoren durchgeführt werden. Die Umsetzung kann dabei in allen Fällen diskontinuierlich oder kontinuierlich ausgeführt werden.

Gemäß einer Ausführungsform des erfindungsgemäßen Verfahrens wird gegebenenfalls am aromatischen Kern in o- oder m-Position mit 1 bis 4 Substituenten, ausgewählt aus C₁₋₂₀-, vorzugsweise C₁₋₁₀-, besonders bevorzugt C₁₋₆-, insbesondere C₁₋₃-Alkylresten und Halogenatomen, vorzugsweise Fluor-, Chlor- oder Bromatomen, insbesondere Chloratomen substituiertes Anilin mit Formaldehyd oder Formaldehyd-Vorläufern umgesetzt.

Vorzugsweise liegen im gegebenenfalls substituierten Anilin 0 bis 2, besonders bevorzugt keiner oder ein Substituent vor. Liegt ein Substituent vor, so ist es vorzugsweise ein Alkylrest, insbesondere ein Methyl-, Ethyl- oder Propylrest.. Dieser Substituent liegt vorzugsweise in der o-Position vor. Bevorzugt eingesetzte Verbindungen sind Anilin oder o-Toluidin. Das gegebenenfalls substituierte Anilin wird mit Formaldehyd oder Formaldehyd-Vorläufern umgesetzt. Als Formaldehyd-Vorläufer dienen solche Verbindungen, die unter den Reaktionsbedingungen Formaldehyd freisetzen. Beispiele hierfür sind Paraformaldehyd und Trioxan.

Die Umsetzung kann dabei direkt in Gegenwart des erfindungsgemäßen Katalysators durchgeführt werden, d.h. der Katalysator wird bereits direkt zu Beginn der Umsetzung zugefügt. Dabei können beispielsweise das gegebenenfalls substituierte Anilin gemeinsam mit dem Katalysator vorgelegt und der Formaldehyd gasförmig oder als wäßrige Lösung oder als Formaldehyd-Vorläufer zudosiert werden. Die Umsetzung kann auch durchgeführt werden, indem der Formaldehyd oder der Formaldehyd-Vorläufer mit dem gegebenenfalls substituierten Anilin gemeinsam vorgelegt und der Katalysator anschließend eingetragen wird. Gemäß dieser weiteren Ausführungsform der Erfindung wird zunächst der Formaldehyd mit dem gegebenenfalls substituierten Anilin in Abwesenheit des Katalysators umgesetzt, wobei sich eine Kondensationsverbindung der allgemeinen Formel (IV) bzw. (V) bildet. Dieses Vorkondensat kann anschließend in Gegenwart des erfindungsgemäßen Katalysators umgesetzt werden, wobei die Umlagerung zu einer Verbindung der allgemeinen Formel (I) eintritt.

Das bei der Bildung des Vorkondensates entstehende Reaktionswasser kann kontinuierlich entfernt werden. Es kann auch am Ende der Umsetzung zur Bildung des Vorkondensats durch Destillation entfernt werden oder im Reaktionsgemisch verbleiben.

Wird ein gegebenenfalls substituiertes Anilin eingesetzt, bei dem nicht beide o-Positionen substituiert sind, so können bei der Isomerisierung neben den 4,4'-Isomeren der Formel (I) auch die 2,4'-Isomeren der Formel (II) und die 2,2'-Isomeren der Formel (III) gebildet werden. Die Erfindung betrifft so auch ein Verfahren, bei dem die aromatischen Kerne in mindestens einer o-Stellung zur Aminogruppe nicht substituiert sind und die aromatischen Polyamingemische ferner Verbindungen der allgemeinen Formeln (II) und /oder (III) enthalten

H₂N-D-CH₂-B-NH₂ (II)

in der D ein 1,2-Phenylenrest und B ein 1,4-Phenylenrest sind, die die vorstehenden Substituenten aufweisen können,

H₂N-D-CH₂-E-NH₂ (III)

in der D und E 1,2-Phenylenreste sind, die die vorstehenden Substituenten aufweisen können.

Durch Verwendung der erfindungsgemäßen Katalysatoren kann der Anteil der Verbindungen der Formel (I) gegenüber dem Anteil der Verbindungen der Formeln (II) und (III) gezielt variiert werden. Verbindungen der Formel (III) werden nur in sehr untergeordnetem Maße gebildet. Das Molverhältnis von Verbindungen der Formel (I) zu Verbindungen der Formel (II) beträgt vorzugsweise mehr als 4, sofern Verbindungen der Formel (II) gebildet werden können.

Die erfindungsgemäße Umsetzung, insbesondere die Umlagerungsreaktion, verläuft vorzugsweise im Temperaturbereich von 20°C bis 200°C, besonders bevorzugt von 100°C bis 150°C. Die Umsetzung kann dabei in Abwesenheit oder in Gegenwart eines Lösungsmittels durchgeführt werden. Als Lösungsmittel kommmen protische Lösungsmittel wie Alkohole, auch Diole wie Glykol, oder aprotische Lösungsmittel wie N-Methylpyrrolidon zum Einsatz. Vorzugsweise wird die Umsetzung in Abwesenheit eines Lösungsmittels durchgeführt.

Die Umsetzung wird üblicherweise drucklos durchgeführt, kann aber auch bei Unterdruck oder Überdruck durchgeführt werden. Die Umsetzungszeit beträgt - je nach Temperatur - vorzugsweise 10 Minuten bis 10 Stunden, besonders bevorzugt 0,5 bis 5 Stunden oder 1 bis 5 Stunden bei diskontinuierlicher Fahrweise. Die eingesetzte Katalysatormenge beträgt dabei 1 bis 50, vorzugsweise 5 bis 40 Gew.-%, bezogen auf das Gewicht des Vorkondensates. Bei kontinuierlicher Fahrweise wird vorzugsweise eine Katalysatorbelastung von 0,1 bis 1 l Ausgangsgemisch/l Katalysator x h gefahren. Das Molverhältnis des gegebenenfalls substituierten Anilins zum Formaldehyd beträgt vorzugsweise 2 bis 50, besonders bevorzugt 2,5 bis 10.

Zur Aufarbeitung wird das Reaktionsgemisch - bei Suspensionsfahrweise nach Abfiltrieren des Katalysatorpulvers - destillativ von gegebenenfalls vorliegendem Lösungsmittel, beziehungsweise nicht umgesetztem, gegebenenfalls substituiertem Anilin, befreit und anschließend vom gegebenenfalls anfallenden hochsiedenden Rückstand abdestilliert. Das hierbei als Destillat erhaltene Polyamingemisch kann direkt in Folgereaktionen, wie einer Phosgenierung oder auch Kernhydrierung, eingesetzt werden.

Nachstehend wird die Erfindung anhand von Beispielen näher erläutert. Dabei bedeuten die angegebenen Teile Gewichtsteile.

### Ton-Katalysator

### Katalysatorherstellung

### Katalysator A

Katalysator A wurde hergestellt aus kommerziell erhältlichem K 10 (säureaktivierter Montmorillonit von Südchemie), der zuvor verstrangt wurde. Eine Lösung aus 100 g Ba(NO₃)₂ in 2 Liter destilliertem Wasser wurde 3 Stunden bei 50 °C über 1200 g der Stränge gepumpt, und diese wurden danach kurz mit destilliertem Wasser gespült. Nach Trocknen bei 120°C für 16 Stunden wurde noch 2 Stunden bei 200°C kalziniert.
Katalysator A enthielt 1,25 % BaO.

### Katalysator B

Katalysator B wurde analog zu Katalysator A hergestellt, nur daß statt Ba(NO₃)₂ eine Mischung verschiedener Nitrate der seltenen Erden eingesetzt wurde.
Katalysator B enthielt 2,7 % CeO₂, 1,48 % La₂O₃, 0,32 % Pr₂O₃ und 1,11 % Nd₂O₃.

### Katalysator C

Katalysator C wurde analog zu Katalysator A hergestellt, nur daß statt Ba(NO₃)₂ Ga(NO₃)₃ · 9H₂O eingesetzt und bei 400°C kalziniert wurde.
Katalysator C enthielt 1,13 % Ga₂O₃.

### Katalysator D

Katalysator D wurde analog zu Katalysator A hergestellt, nur daß statt Ba(NO₃)₂ Sr(NO₃)₂ eingesetzt und bei 400°C kalziniert wurde.
Katalysator D enthielt 0,84 % SrO.

### Katalysator E

Katalysator E wurde analog zu Katalysator A hergestellt, nur daß statt Ba(NO₃)₂ In(NO₃) · 5H₂O eingesetzt und bei 400°C kalziniert wurde.
Katalysator E enthielt 1,57 % In₂O₃.

### Katalysator F

Katalysator E wurde analog zu Katalysator A hergestellt, nur daß statt Ba(NO₃)₂ HfCl₄ eingesetzt und bei 400°C kalziniert wurde.
Katalysator F enthielt 3,3 % HfO₂.

### Katalysator G

Katalysator G wurde hergestellt aus kommerziell erhältlichem K 10 (säureaktivierter Montmorillonit von Südchemie), der zuvor verstrangt wurde. Eine Lösung aus 120 g Re₂O₇ in 4 Liter destilliertem Wasser wurde 3 Stunden bei 50°C über 2000 g der Stränge gepumpt, und diese wurden danach kurz mit destilliertem Wasser gespült. Nach Trocknen bei 120°C für 16 Stunden wurde noch 2 Stunden bei 400°C kalziniert.
Katalysator G enthielt 0,65 % Re₂O₇.

### Katalysator H

Katalysator H wurde hergestellt aus kommerziell erhältlichem K 10 (säureaktivierter Montmorillonit von Südchemie), der zuvor verstrangt wurde. Eine Lösung aus 400g Re₂O₇ in 3 Liter destilliertem Wasser wurde 6 Stunden bei 50°C über 2000 g der Stränge gepumpt, und diese wurden danach kurz mit destilliertem Wasser gespült. Nach Trocknen bei 120°C für 16 Stunden wurde noch 2 Stunden bei 400°C kalziniert.
Katalysator H enthielt 0,45 % Re₂O₇.

### Reaktion zu Polyaminen

160 g (1,5 Mol) o-Toluidin wurden bei Raumtemperatur mit 7,5 g (0,25 Mol) Formaldehyd (als 37%ige wäßrige Lösung) versetzt, anschließend wurde über einen Wasserauskreiser bei 150°C das Wasser ausgekreist. Die erhaltene Lösung dieses Vorkondensats in Toluidin wurde direkt für die Umsetzung mit den Katalysatoren b) eingesetzt.

Hierzu wurden jeweils 40 Gew.-% (bezogen auf das Anlagerungsprodukt aus Formaldehyd und zwei Molekülen Toluidin) Katalysator b) in gepulverter Form hinzugegeben und die Mischung unter Rühren auf 100°C erwärmt. Die Ergebnisse mit den verschiedenen Katalysatoren nach unterschiedlichen Reaktionszeiten sind in Tabelle 1 dargestellt. Ein Gehalt von etwa 30 % Toluidinbase entspricht hierbei der maximal möglichen Ausbeute. Das angegebene Isomerenverhältnis gibt den Anteil des 4,4'-Isomeren relativ zu dem 2,4'-Isomeren wieder.

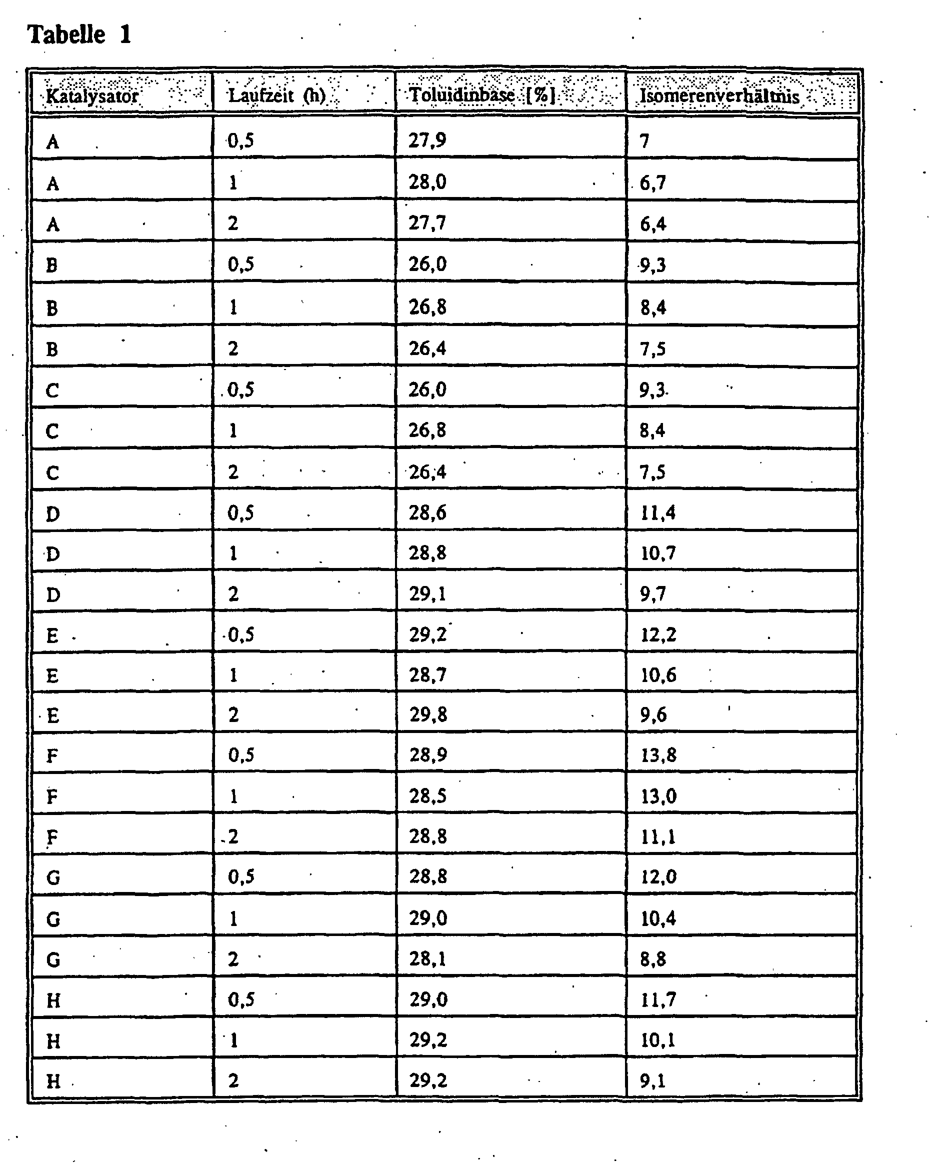

### Schichtsilikat-Katalysator

### Katalysatorherstellung

### Katalysator A (Vergleichsbeispiel)

Katalysator A war ein kommerziell erhältlicher säureaktivierter Montmorillonit von Südchemie (Typ: KSF) ohne acide Zentren unterhalb von pKₛ=1,5.

### Katalysator B

Katalysator B war ein kommerziell erhältlicher säureaktivierter Montmorillonit von Südchemie (Typ: K 10) mit einer Acidität von 0,061 mmol g⁻¹ unterhalb von pKₛ = 1,5.

### Katalysator C

Katalysator C war ein kommerziell erhältlicher Attapulgit von Floridin.

### Katalysator D (Vergleichsbeispiel)

Katalysator D wurde durch Säureaktivierung (0,5 M H₂SO₄, 12 h 50°C) eines kommerziell erhältlichen Sepioliten von Oxymine erhalten. Er besaß keine Acicidät unterhalb von pKₛ = 1,5.

### Katalysator E (Vergleichsbeispiel)

Katalysator E wurde durch Säureaktivierung (1 M HCl, 24 h 50°C) eines kommerziell erhältlichen Sepioliten von Sobrep erhalten. Er besaß keine Acidität unterhalb von pKₛ = 1,5.

### Katalysator F

Katalysator F wurde durch Säureaktivierung (1 M HCl, 4 h 50°C) eines kommerziell erhältlichen Montmorilloniten (Terrana D von Südchemie) erhalten. Er besaß eine Acidität von 0,090 mmol g⁻¹ unterhalb von pKₛ = 1,5.

### Katalysator G (Vergleichsbeispiel)

Katalysator G wurde durch Säureaktivierung (0,5 M H₂SO₄, 6h 50°C) eines kommerziell erhältlichen synthetischen Hectoriten (Laponit RD von Laporte) erhalten. Er besaß eine Acidität von 0,021 mmol g⁻¹ unterhalb von PKₛ = 1,5.

### Katalysator H

Katalysator H wurde durch Säureaktivierung (0,5 M H₃PO₄, 12 h 50°C) eines kommerziell erhältlichen Montmorilloniten (Terrana D von Südchemie) erhalten. Er besaß eine Acidität von 0,10 mmol g⁻¹ unterhalb von pKₛ = 1,5.

### Katalysator I

Katalysator I wurde durch Säureaktivierung (0,5 M H₂SO₄, 6h 100°C) eines kommerziell erhältlichen Montmorilloniten (Terrana D von Südchemie) erhalten. Er besaß eine Acidität von 0,10 mmol g⁻¹ unterhalb von pKₛ = 1,5.

### Katalysator J (Vergleichsbeispiel)

Katalysator J wurde durch Säureaktivierung (1 M HCl, 24 h 50°C) eines kommerziell erhältlichen Vermiculiten (Mikro von Ziegler) erhalten. Er besaß eine Acidität von 0,031 mmol g⁻¹ unterhalb von pKₛ = 1,5.

### Katalysator K (Vergleichsbeispiel)

Katalysator K war ein kommerziell erhältlicher Sepiolit von Sobrep. Er besaß eine Acidität von 0,004 mmol g⁻¹ unterhalb von pKₛ = 1,5.

### Katalysator L (Vergleichsbeispiel)

Katalysator L war ein kommerziell erhältlicher synthetischer Hectorit (Laponit RD von Laporte). Er besaß keine Acidität unterhalb von pKₛ = 1,5. Das Beispiel zeigt, daß Katalysatoren mit unzureichender Acidität zwar Umsätze in ausreichender Höhe erreichen können, sich aber gleichzeitig das Isomerenverhältnis verschlechtert.

### Katalysator M

Katalysator M war ein kommerziell erhältlicher Sepiolit von Oxymine. Er besaß eine Acidität von 0,05 mmol g⁻¹ unterhalb von pkₛ=1,5.

### Katalysator N (Vergleichsbeispiel)

Katalysator N war eine kommerziell erhätlicher Hectorit (Bentone MA von Rheox). Er besaß keine Acidität unterhalb von pKₛ=1,5. Das Beispiel zeigt, daß Katalysatoren mit unzureichender Acidität zwar Umsätze in ausreichender Höhe erreichen können, sich aber gleichzeitig das Isomerenverhältnis verschlechtert.

### Reaktion zu Polyaminen

160 g (1,5 Mol) o-Toluidin wurden bei Raumtemperatur mit 7,5 g (0,25 Mol) Formaldehyd (als 37 %ige wäßrige Lösung) versetzt, anschließend wurde über einen Wasserauskreiser bei 150°C das Wasser ausgekreist. Die erhaltene Lösung dieses Vorkondensats in Toluidin wurde direkt für die Umsetzung mit den Katalysatoren c) eingesetzt.

Hierzu wurden jeweils 40 Gew.-% (bezogen auf das Anlagerungsprodukt aus Formaldehyd und zwei Molekülen Toluidin) Katalysator c) in Pulver-Form hinzugegeben und die Mischung unter Rühren auf 100°C erwärmt. Die Ergebnisse mit den verschiedenen Katalysatoren c) nach unterschiedlichen Reaktionszeiten sind in Tabelle 1 dargestellt. Ein Gehalt von etwa 30 % Toluidinbase entspricht hierbei der maximal möglichen Ausbeute. Das angegebene Isomerenverhältnis gibt den Anteil des 4,4'-Isomeren relativ zu dem 2,4'-Isomeren wieder.

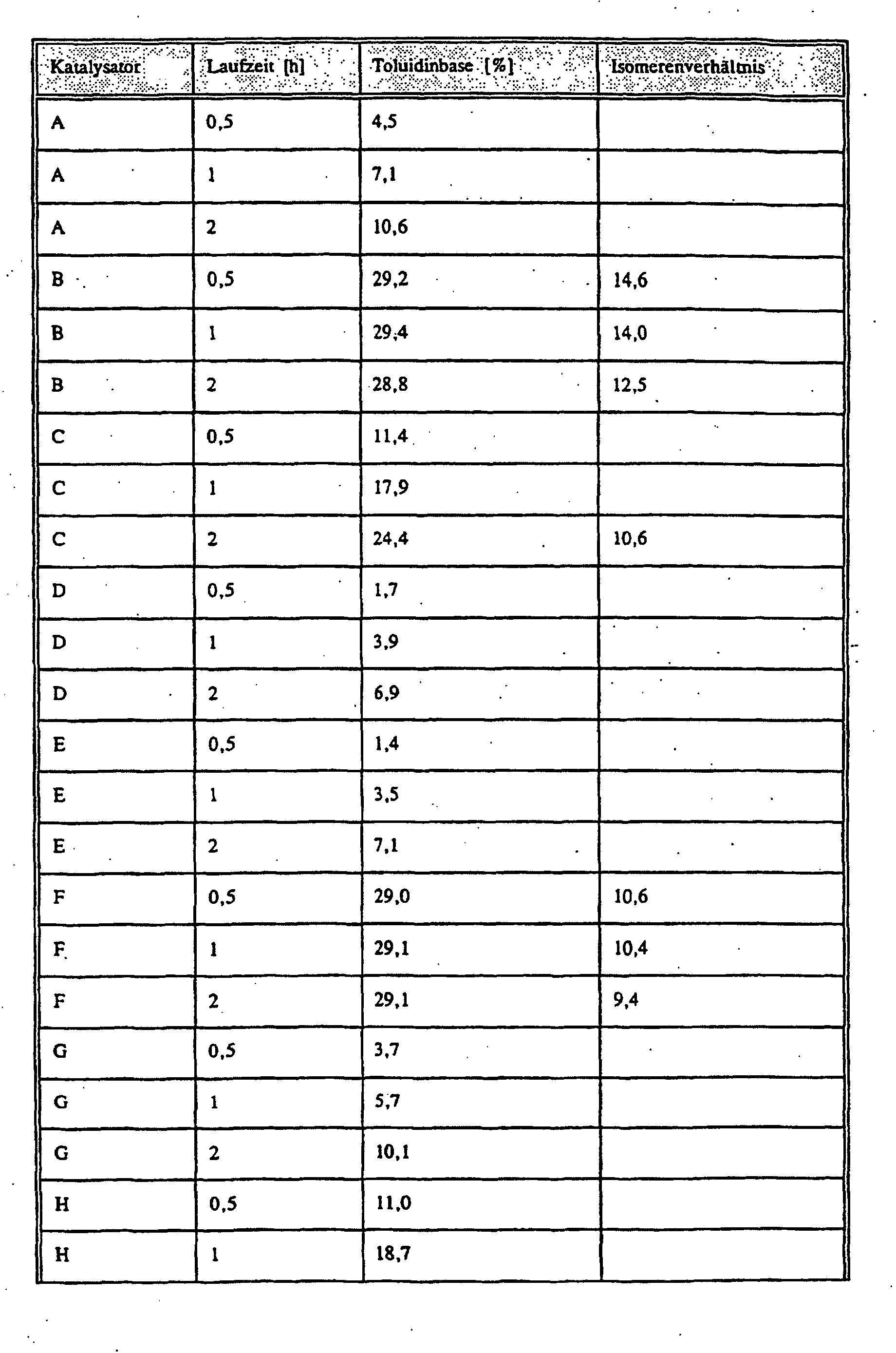

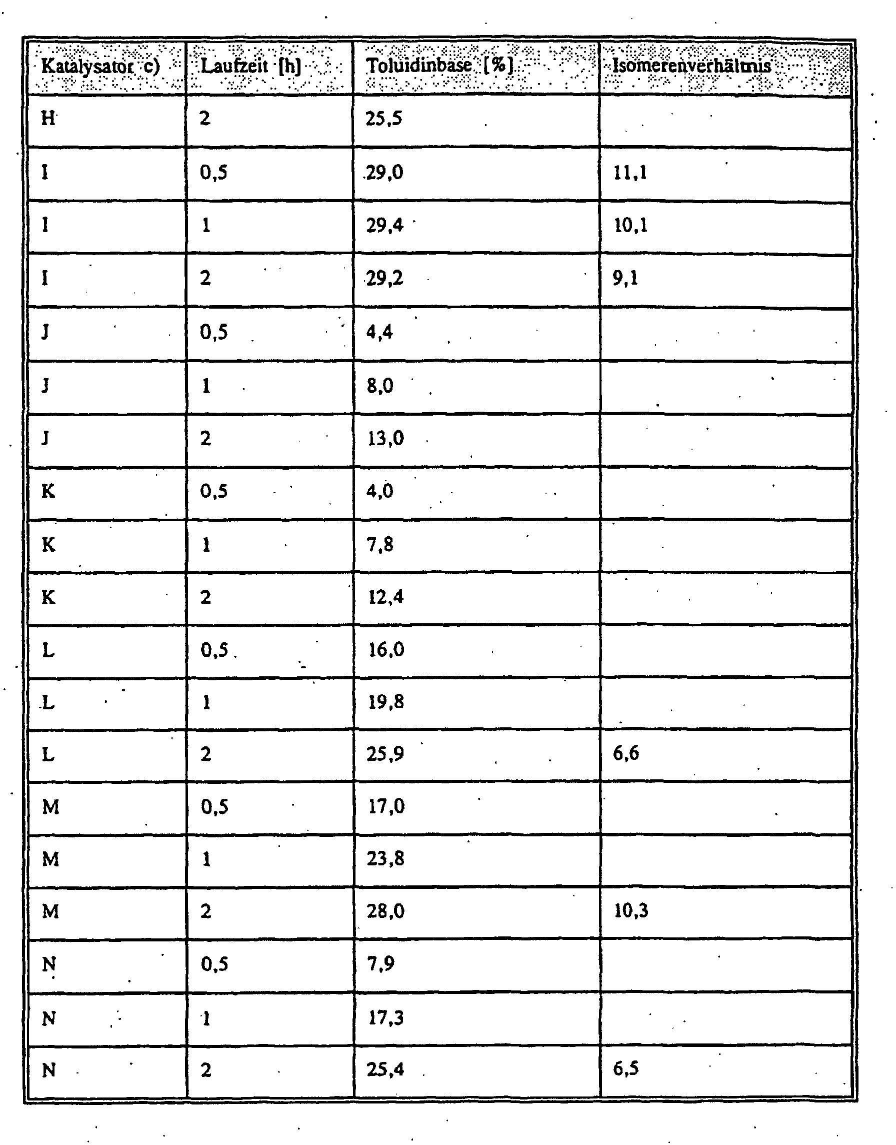

## Patentansprüche

1. Verfahren zur Herstellung von aromatischen Polyamingemischen, die Verbindungen der allgemeinen Formel (I) enthalten,
H₂N-A-CH₂-B-NH₂ (I)
in der A und B 1,4-Phenylenreste sind, die jeweils unabhängig 1 bis 4 Substituenten, ausgewählt aus C₁₋₂₀-Alkylresten und Halogenatomen, aufweisen können,
durch Umsetzung einer Verbindung der allgemeinen Formel (IV),
H-A-NH-CH₂-HN-B-H (IV)
und/oder einer Verbindung der allgemeinen Formel (V)
H-A-NH-CH₂-B-NH₂ (V)
wobei A und B wie vorstehend definiert substituiert sind,
bei einer Temperatur im Bereich von 20°C bis 200°C in Gegenwart eines heterogenen anorganischen Katalysators, der ausgewählt ist aus einem Ton, der mit mindestens einem Oxid von Elementen der Gruppen 2 bis 13 oder der Lanthaniden des Periodensystems der Elemente durch Imprägnierung oder über einen Ionenaustausch dotiert ist und säureaktiviert sein kann; oder einem oder mehreren gegebenenfalls säureaktivierten Schichtsilikaten, die eine Acidität unterhalb von pKₛ=1,5 von mehr als 0,05 mmol/g Katalysator besitzen.

2. Verfahren nach Anspruch 1, **dadurch gekennzeichnet, daß** die Verbindung der allgemeinen Formeln (IV) oder (V) durch Umsetzung von gegebenenfalls am aromatischen Kern in o- oder m- Position mit 1 bis 4 Substituenten, ausgewählt aus C₁₋₂₀-Alkylresten oder Halogenatomen, substituiertem Anilin mit Formaldehyd oder Formaldehyd-Vorläufern erhalten wird.

3. Verfahren nach Anspruch 1 zur Herstellung von aromatischen Polyamingemischen, die Verbindungen der allgemeinen Formel (I) enthalten
H₂N-A-CH₂-B-NH₂ (I)
in der A und B 1,4-Phenylenreste sind, die jeweils unabhängig 1 bis 4 Substituenten, ausgewählt aus C₁₋₂₀-Alkylresten und Halogenatomen, aufweisen können,
durch Umsetzung von gegebenenfalls am aromatischen Kern in o- oder m- Position mit 1 bis 4 Substituenten, ausgewählt aus C₁₋₂₀-Alkylresten und Halogenatomen, substituierten Anilinen mit Formaldehyd oder Formaldehyd-Vorläufern bei einer Temperatur im Bereich von 20°C bis 200°C in Gegenwart eines heterogenen Katalysators, der ausgewählt ist aus einem Ton, der mit mindestens einem Oxid von Elementen der Gruppen 2 bis 13 oder der Lanthaniden des Periodensystems der Elemente durch Imprägnierung oder über einen Ionenaustausch dotiert ist und säureaktiviert sein kann; oder einem oder mehreren gegebenenfalls säureaktivierten Schichtsilikaten, die eine Acidität unterhalb von pKₛ=1,5 von mehr als 0,05 mmol/g Katalysator besitzen.

4. Verfahren nach Anspruch 2 oder 3, **dadurch gekennzeichnet, daß** das bei der Umsetzung entstehende Wasser kontinuierlich entfernt wird.

5. Verfahren nach einem der Ansprüche 1 bis 4, **dadurch gekennzeichnet, daß** die aromatischen Kerne in mindestens einer o-Stellung zur Aminogruppe nicht substituiert sind und die aromatischen Polyamingemische ferner Verbindungen der allgemeinen Formeln (II) und/oder (III) enthalten
H₂N-D-CH₂-B-NH₂ (II)
in der D ein 1,2-Phenylenrest und B ein 1,4-Phenylenrest sind, die durch Substituenten, wie sie in Anspruch 3 definiert sind, substituiert sein können,
H₂N-D-CH₂-E-NH₂ (III)
in der D und E 1,2-Phenylenreste sind, die durch Substituenten, wie sie in Anspruch 3 definiert sind, substituiert sein können.

6. Verfahren nach Anspruch 2, 3 oder 4, **dadurch gekennzeichnet, daß** Anilin oder o-Toluidin eingesetzt werden.

7. Verfahren nach einem der Ansprüche 1 bis 6, **dadurch gekennzeichnet, daß** als Katalysator
ein oder mehrere Tone eingesetzt werden, die mit mindestens einem Oxid von Elementen der Gruppen 2, 3, 4, 13 oder der Lanthaniden des Periodensystems der Elemente dotiert sind, wobei der Katalysator säureaktiviert sein kann; oder
ein oder mehrere gegebenenfalls säureaktivierte Schichtsilikate aus der Gruppe der Saponite, Hectorite, Montmorillonite, Sepiolite oder Attapulgite eingesetzt werden.

8. Verfahren nach einem der Ansprüche 1 bis 7, **dadurch gekennzeichnet, daß** der Katalysator 0,1 bis 20 Gew.-%, bezogen auf das Gesamtgewicht des Katalysators, an Dotierung enthält, gerechnet als Metalloxid des stabilsten Oxids nach Glühen bei 900°C.

9. Verfahren nach einem der Ansprüche 1 bis 8, **dadurch gekennzeichnet, daß** die Verbindung der Formel (I) als Hauptprodukt gebildet wird.

10. Verfahren nach einem der Ansprüche 1 bis 9, **dadurch gekennzeichnet, daß** die Umsetzung in Abwesenheit eines Lösungsmittels durchgeführt wird.

11. Verwendung von heterogenen anorganischen Katalysatoren, die ausgewählt sind aus
einem Ton, der mit mindestens einem Oxid eines Elements der Gruppen 2 bis 13 oder der Lanthaniden des Periodensystems der Elemente durch Imprägnierung oder über einen Ionenaustausch dotiert ist und säureaktiviert sein kann; oder
einem oder mehreren Schichtsilikaten, die eine Acidität unterhalb von pKₛ = 1,5 von mehr als 0,05 mmol/g Katalysator besitzen und säureaktiviert sein können,
bei der Herstellung von aromatischen Polyamingemischen, wie sie in Anspruch 1 oder 5 definiert sind.

## Claims

1. A process for preparing aromatic polyamine mixtures comprising compounds of the formula (I),
H₂N-A-CH₂-B-NH₂ (I)
where A and B are 1,4-phenylene radicals which can each independently bear from 1 to 4 substituents selected from the group consisting of C₁₋₂₀-alkyl radicals and halogen atoms,
by reacting a compound of the formula (IV)
H-A-NH-CH₂-HN-B-H (IV)
and/or a compound of the formula (V)
H-A-NH-CH₂-B-NH₂ (V)
where A and B are substituted as defined above,
at from 20°C to 200°C in the presence of a heterogeneous inorganic catalyst which is
a clay which has been doped with at least one oxide of elements of groups 2 to 13 or the lanthanides of the Periodic Table of the Elements by impregnation or ion exchange and may be acid-activated, or
one or more sheet silicates which may be acid-activated and have an acidity below pKₐ = 1.5 of more than 0.05 mmol/g of catalyst.

2. A process as claimed in claim 1, wherein the compound of the formula (IV) or (V) is obtained by reacting unsubstituted aniline or aniline substituted in the o and/or m positions of the aromatic ring by from 1 to 4 substituents selected from the group consisting of C₁₋₂₀-alkyl radicals or halogen atoms with formaldehyde or formaldehyde precursors.

3. A process as claimed in claim 1 for preparing aromatic polyamine mixtures comprising compounds of the formula (I),
H₂N-A-CH₂-B-NH₂ (I)
where A and B are 1,4-phenylene radicals which can each independently bear from 1 to 4 substituents selected from the group consisting of C₁₋₂₀-alkyl radicals and halogen atoms,
by reacting unsubstituted anilines or anilines substituted in the o- and/or m-positions of the aromatic ring by from 1 to 4 substituents selected from the group consisting of C₁₋₂₀-alkyl radicals and halogen atoms with formaldehyde or formaldehyde precursors at from 20°C to 200°C in the presence of a heterogeneous catalyst which is
a clay which has been doped with at least one oxide of elements of groups 2 to 13 or the lanthanides of the Periodic Table of the Elements by impregnation or ion exchange and may be acid-activated, or
one or more sheet silicates which may be acid-activated and have an acidity below pKₐ = 1.5 of more than 0.05 mmol/g of catalyst.

4. A process as claimed in claim 2 or 3, wherein the water formed in the reaction is removed continuously.

5. A process as claimed in any of claims 1 to 4, wherein the aromatic rings are unsubstituted in at least one o position to the amino group and the aromatic polyamine mixtures further comprise compounds of the formulae (II) and/or (III),
H₂N-D-CH₂-B-NH₂ (II)
where D is a 1,2-phenylene radical and B is a 1,4-phenylene radical, each of which may be substituted by substituents as defined in claim 3,
H₂N-D-CH₂-E-NH₂ (III)
where D and E are 1,2-phenylene radicals which may be substituted by substituents as defined in claim 3.

6. A process as claimed in claim 2, 3 or 4, wherein aniline or o-toludine are used.

7. A process as claimed in any of claims 1 to 6, wherein the catalyst used is
one or more clays which have been doped with at least one oxide of elements of groups 2, 3, 4, 13 or the lanthanides of the Periodic Table of the Elements, where the catalyst may be acid-activated, or
one or more sheet silicates which may be acid-activated and are selected from the group consisting of saponites, hectorites, montmorillonites, sepiolites or attapulgites.

8. A process as claimed in any of claims 1 to 7, wherein
the catalyst contains from 0.1 to 20% by weight, based on the total weight of the catalyst, of dopant, calculated as metal oxide of the most stable oxide after ignition at 900°C.

9. A process as claimed in any of claims 1 to 8, wherein the compound of the formula (I) is formed as main product.

10. A process as claimed in any of claims 1 to 9, wherein the reaction is carried out in the absence of a solvent.

11. The use of a heterogeneous inorganic catalyst which is
a clay which has been doped with at least one oxide of an element of groups 2 to 13 or the lanthanides of the Periodic Table of the Elements by impregnation or ion exchange and may be acid-activated, or
one or more sheet silicates which have an acidity below pKₐ = 1.5 of more than 0.05 mmol/g of catalyst and may be acid-activated,
in the preparation of aromatic polyamine mixtures as are defined in claim 1 or 5.

## Revendications

1. Procédé pour la préparation de mélanges de polyamines aromatiques, contenant des composés de la formule générale (I),
H₂N-A-CH₂-B-NH₂ (I)
dans laquelle A et B sont des restes 1,4-phénylène, qui peuvent à chaque fois présenter indépendamment l'un de l'autre de 1 à 4 substituants, choisis parmi des restes alkyle en C₁₋₂₀ et des atomes d'halogène,
par réaction d'un composé de la formule générale (IV)
H-A-NH-CH₂-HN-B-H (IV)
et/ou d'un composé de la formule générale (V)
H-A-NH-CH₂-B-NH₂ (V)
où A et B sont substitués comme défini plus haut,
à une température de l'ordre de 20°C à 200°C, en présence d'un catalyseur inorganique hétérogène choisi parmi
- une argile, dopée par au moins un oxyde d'éléments des groupes 2 à 13 ou des lanthanides du système périodique des éléments, par imprégnation ou via un échange d'ions, et qui peut être activée par un acide, ou
- un ou plusieurs phyllosilicates éventuellement activés par un acide, et qui possèdent une acidité au-dessous de pKₛ = 1,5 de plus de 0,05 mmole/g de catalyseur.

2. Procédé selon la revendication 1, **caractérisé en ce que** le composé de formule générale (IV) ou (V) est obtenu par réaction d'aniline éventuellement substituée sur le noyau aromatique, en position ortho ou méta, par 1 à 4 substituants, choisis parmi des restes alkyle en C₁₋₂₀ ou des atomes d'halogène, avec du formaldéhyde ou des précurseurs de formaldéhyde.

3. Procédé selon la revendication 1 pour la préparation de mélanges de polyamines aromatiques, contenant des composés de la formule générale (I)
H₂N-A-CH₂-B-NH₂ (I)
dans laquelle A et B sont des restes 1,4-phénylène, qui peuvent à chaque fois présenter indépendamment l'un de l'autre de 1 à 4 substituants, choisis parmi des restes alkyle en C₁₋₂₀ et des atomes d'halogène,
par réaction d'aniline éventuellement substituée sur le noyau aromatique, en position ortho ou méta, par 1 à 4 substituants, choisis parmi des restes alkyle en C₁₋₂₀ ou des atomes d'halogène, avec du formaldéhyde ou des précurseurs de formaldéhyde, à une température de l'ordre de 20°C à 200°C, en présence d'un catalyseur hétérogène, choisi parmi
- une argile, dopée par au moins un oxyde d'éléments des groupes 2 à 13 ou des lanthanides du système périodique des éléments, par imprégnation ou via un échange d'ions, et qui peut être activée par un acide, ou
- un ou plusieurs phyllosilicates éventuellement activés par un acide, et qui possèdent une acidité au-dessous de pKₛ = 1,5 de plus de 0,05 mmole/g de catalyseur.

4. Procédé selon la revendication 2 ou 3, **caractérisé en ce que** l'eau formée par la réaction est éliminée en continu.

5. Procédé selon l'une quelconque des revendications 1 à 4, **caractérisé en ce que** les noyaux aromatiques ne sont pas substitués dans au moins une position ortho par rapport au groupe amino et que les mélanges de polyamines aromatiques contiennent en outre des composés des formules générales (II) et/ou (III)
H₂N-D-CH₂-B-NH₂ (II)
dans laquelle D est un reste 1,2-phénylène et B un reste 1,4-phénylène, ces restes pouvant être substitués par des substituants tels que définis à la revendication 3,
H₂N-D-CH₂-E-NH₂ (III)
dans laquelle D et E sont des restes 1,2-phénylène qui peuvent être substitués par des substituants tels que définis à la revendication 3.

6. Procédé selon la revendication 2, 3 ou 4, **caractérisé en ce que** l'on met en oeuvre de l'aniline ou de l'o-toluidine.

7. Procédé selon l'une quelconque des revendications 1 à 6, **caractérisé en ce que** l'on utilise comme catalyseur
- une ou plusieurs argiles dopées par au moins un oxyde d'éléments des groupes 2, 3, 4, 13 ou des lanthanides du système périodique des éléments, le catalyseur pouvant être activé par un acide, ou
- un ou plusieurs phyllosilicates éventuellement activés par un acide, du groupe des saponites, des hectorites, des montmorillonites, des sépiolites ou des attapulgites.

8. Procédé selon l'une quelconque des revendications 1 à 7, **caractérisé en ce que**
- le catalyseur contient de 0,1 à 20% en poids, par rapport au poids total du catalyseur, de dopage, calculé en oxyde métallique de l'oxyde le plus stable après calcination à 900°C.

9. Procédé selon l'une quelconque des revendications 1 à 8, **caractérisé en ce que** le composé de formule (I) est formé en tant que principal produit.

10. Procédé selon l'une quelconque des revendications 1 à 9, **caractérisé en ce que** la réaction est entreprise en l'absence de solvant.

11. Utilisation de catalyseurs inorganiques hétérogènes, choisis parmi
- une argile, dopée par au moins un oxyde d'éléments des groupes 2 à 13 ou des lanthanides du système périodique des éléments, par imprégnation ou via un échange d'ions, et qui peut être activée par un acide, ou
- un ou plusieurs phyllosilicates qui possèdent une acidité au-dessous de pKₛ = 1,5 de plus de 0,05 mmole/g de catalyseur, et qui peuvent être activés par un acide,
pour la préparation de mélanges de polyamines aromatiques, comme définies aux revendications 1 ou 5.
